# EUROPEAN PATENT APPLICATION

(11) **EP 4 763 860 A1**
(43) Date of publication of application: **24.06.2026**
(21) Application number: 24222426.9
(22) Date of filing: 20.12.2024
(51) Int. Cl.: C07K 16/00

(54) **METHOD FOR VHH LIBRARY CONSTRUCTION**

(71) Applicant: Biotalys NV, 9051 Gent (BE)
(72) Inventor: GRIEP, Remko, Albert, Nijmegen (NL)
(74) Representative: Gevers Patents

(57) **Abstract**

The invention relates to an improved and simplified method for constructing VHH antibody libraries. The present invention provides a method for constructing a VHH antibody library comprising performing DNA amplification on a cDNA preparation from peripheral blood mononuclear cells of a *Camelidae* species. Hereby amplifying the variable domains and constant domains of the heavy chain only antibodies (HCAbs) whilst blocking amplification of the variable domains and constant domains of conventional heavy and light chain antibodies present in the cDNA preparation, wherein blocking occurs using at least one blocking oligonucleotide. The invention further relates to a kit of parts and a method of obtaining one or more VHH binding to an antigen.

## Description

### Field of the invention

The invention relates to an improved and simplified method for constructing VHH antibody libraries. The present invention provides a method for constructing a VHH antibody library comprising performing DNA amplification on a cDNA preparation from peripheral blood mononuclear cells of a *Camelidae* species. Hereby amplifying the variable domains (VHH) and constant domains (CH) of the heavy chain only antibodies (HCAbs) whilst blocking amplification of the variable domains (VH) and constant domains (CH) of conventional heavy and light chain containing antibodies present in the cDNA preparation, wherein blocking occurs using at least one blocking oligonucleotide. The invention further relates to a kit of parts and a method of obtaining one or more VHH binding to an antigen.

The present invention therefore relates to VHH antibodies, and concerns methods, uses, and kits associated with preparing VHH antibody libraries. Specifically, the invention relates to nucleic acid amplification based methods for preparing libraries of VHH antibodies.

### Background

Discovery of heavy chain only antibodies (HCAbs) and particularly the isolation of the variable region of a heavy chain only antibody (VHH or VHH antibody) has paved the way for various innovative advancements. These antibodies, which are devoid of light chains present in conventional antibodies, are being used in different fields, such as diagnostics and therapeutics, owing to distinctive features including their compact size, better tissue absorption, and robust binding capabilities. One prominent source of VHH antibodies are members of the *Camelidae* family, especially llamas.

In addition to the HCAbs, *Camelidae* species also produce conventional antibodies (such as IgG antibodies), having both the full-length heavy-chain and light-chain variable (VH and VL) and constant (CH and CL) domains. HCAbs consist of the following domains (From the N to C terminus): a VHH domain, a hinge domain a CH2 domain and a CH3 domain (depicted herein as VHH-CH2-CH3). The heavy chain of a conventional antibody consists of the following domains in order: a VHH domain, a CH1 domain, a hinge domain, a CH2 domain and a CH3 domain (depicted herein as VH-CH1-CH2-CH3). Isolation of VHH sequences from immunized camelids is generally performed by mRNA extraction from lymphocytes and conversion to cDNA. Thereafter, the heavy chain region is amplified by PCR using primers that bind to conserved nucleotide sequences in or flanking the VHH domain, e.g. in the framework region of FR1 of the VHH on the 5'end and in the CH2 or CH3 domains downstream of the VHH domain (as heavy-chain only antibodies do not comprise a CH1 domain). This results in the amplification of cDNA encoding the VHH domain, the hinge domain, the CH2 domain or part thereof and optionally the CH3 domain or part thereof of heavy-chain only antibodies (depicted herein as VHH-CH2), but will also amplify the VH domain, CH1 domain, hinge domain, the -CH2 domain or part thereof and optionally the CH3 domain or part thereof from conventional antibodies (depicted herein as VH-CH1-CH2).

Different methods have been reported to selectively separate and construct a VHH antibody library from mixtures which also comprise the conventional antibody sequences. For example, nested PCR approach in combination with gel purification is one such method, which relies on the difference in size between the two types of antibody sequences. Since the cDNA sequences derived from the VHH antibodies are shorter than those derived from the conventional antibodies, this method relies on first separating the two types of sequences using gel electrophoresis. Thereafter, the VHH-CH2 DNA amplification product can be extracted from the gel and undergo nested PCR to selectively amplify the VHH antibody sequence of interest (Nguyen VK., Adv Immunol, 2001). However, this method has multiple drawbacks. For instance, the undesirable amplification of conventional antibody sequences depletes essential PCR reagents, which hinders the amplification of VHH antibody sequence. Additionally, the essential step of extraction of DNA from the gel is not only laborious but also quite inefficient, and results in loss of DNA which could be used for the subsequent nested PCR to generate libraries and may even lead to the loss of particular VHH sequences from the original sample. Moreover, there is a risk of contamination from other DNA sequences in the gel electrophoresis extraction step.

Kastelic et al. reported another PCR-based method for co-amplification of heavy-chain variable domains from both HCAbs and conventional antibodies, which comprises only a single-step PCR using FR1 and FR4 specific primers amplifying both VH and VHH domains and omits the elimination of the co-amplified VH sequences (Kastelic D. et al., J Immunol Methods, 2009). However, this strategy is not considered ideal because the heavy-chain variable domains of conventional antibodies can be sticky if their counterpart light-chain variable domains are not present, thereby complicating separation and enrichment of target VHH antibodies from the mixture (Muyldermans S., FEBS J., 2021).

Therefore, their remains a need to improve the reliability, selectivity, and efficiency of preparing VHH antibody libraries.

### Summary of the invention

The present inventors have developed an improved and simplified method for constructing VHH antibody libraries. The method of the current invention does not require a gel purification or other separation step to first separate the VHH-CH2 amplicon from the VH-CH1-CH2 amplicon, reducing both the potential loss of VHH-CH2 amplicons as well as simplifying the process. Furthermore by channelling more PCR resources to the amplification of the VHH-CH2 domain, a more efficient amplification occurs, leading to significantly improved PCR yields which finally results in VHH antibody libraries having a higher sequence diversity.

Therefore, in a first aspect, the present invention provides a method for constructing a VHH antibody library comprising the steps of (a) providing a cDNA preparation derived from an RNA extract from peripheral blood mononuclear cells of a *Camelidae* species; and (b) performing DNA amplification of VHH-CH2 encoding regions present in the cDNA preparation, whilst blocking DNA amplification of VH-CH1-CH2 encoding regions present in the cDNA preparation, (i) wherein DNA amplification of VHH-CH2 encoding regions is initiated by a primer pair comprising a forward and a reverse primer, where the forward primer hybridizes to and/or upstream of the FR1 domain of the VHH-CH2 and VH-CH1-CH2 encoding regions and where the reverse primer hybridizes to and/or downstream of the CH2 domain of the VHH-CH2 and VH-CH1-CH2 encoding regions, thereby providing VHH-CH2 encoding amplicons and (ii) wherein DNA amplification of VH-CH1-CH2 encoding regions is blocked by at least one blocking oligonucleotide.

In a second aspect, the current invention provides a kit of parts comprising (i) a primer pair suitable to amplify a VHH-CH2 and VH-CH1-CH2 encoding regions in a cDNA preparation derived from an RNA extract from peripheral blood mononuclear cells of a *Camelidae* species; the primer pair comprising at least one forward and a reverse primer, where the forward primer has the ability to hybridize to and/or upstream of the FR1 domain of the VHH-CH2 and VH-CH1-CH2 encoding regions and where the reverse primer has the ability to hybridize to and/or downstream of the CH2 domain of the VHH-CH2 and VH-CH1-CH2 encoding regions; and (ii) a blocking oligonucleotide that is adapted to block DNA amplification of the VH-CH1-CH2 encoding regions by the primer pair, In particular a blocking oligonucleotide that hybridizes to a VH-CH1-CH2 encoding region but not a VHH-CH2 encoding region.

In a third aspect, the current invention further provides a method of obtaining one or more VHH binding to an antigen, comprising the steps of (i) providing the VHH library obtained by the method of the current invention or the VHH antibody library obtained using the kit according to the current invention; (ii) expressing said VHH antibody library; (iii) screening said VHH antibody library for one or more VHH binding to said antigen; and (iv) isolating said one or more VHH.

### Brief description of the drawings

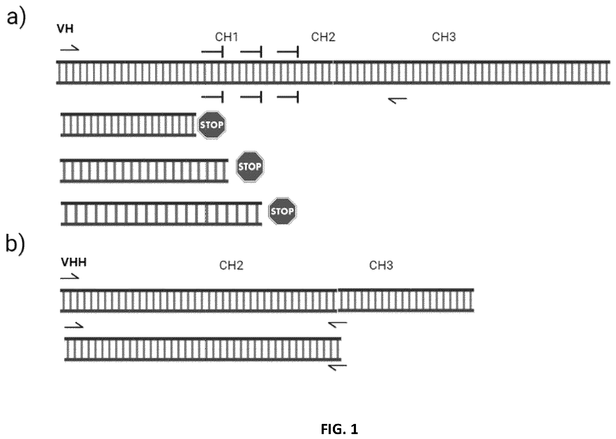

**Figure 1** provides a schematic representation of the mode of action of the inhibition of VH amplification via blocked oligos.

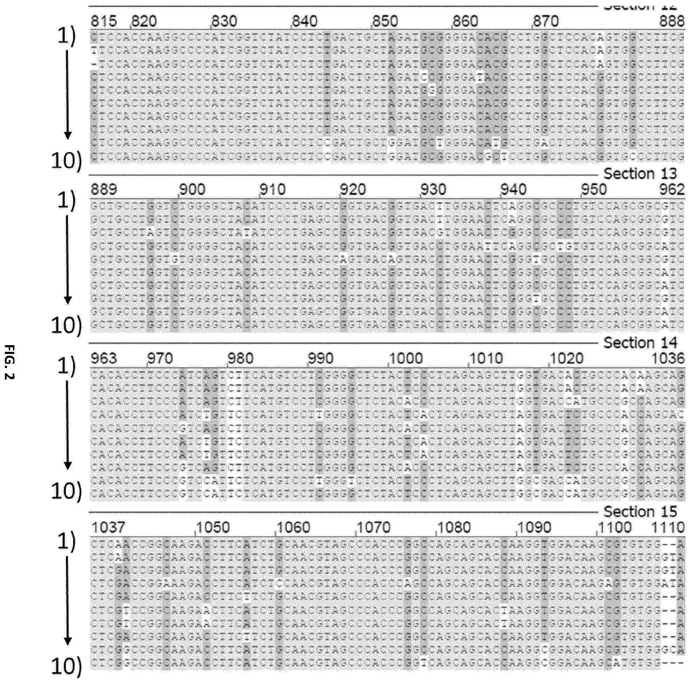

**Figure 2** sets out the alignment of CH1 regions from varia *Camelidae* according to SEQ ID Nos: 6 to 15. In following order from number 1 to 10: 1. D10 *Lama glama* igG1b (SEQ ID NO: 6), 2. AF305955 *Lama glama* IgG1b (SEQ ID NO: 7), 3. AM773729 *Vicugna pacos* IgG1a (SEQ ID NO: 8), 4. AJ131945 *Camelus dromedarius* igG1b (SEQ ID NO: 11), 5. HE653081 *camelus dromedarius* IgG1b (SEQ ID NO: 10), 6. HE653043 *camelus dromedarius* IgG1a (SEQ ID NO: 9), 7. HE653055 *Camelus bacterianus* IgG1a (SEQ ID NO: 12), 8. HE653094 *Camelus bacterianus* IgG1b (SEQ ID NO: 13), 9. XR_004318543 *Camelus bacterianus* (SEQ ID NO: 14), 10. XR_00413296 *Camelus bacterianus* (SEQ ID NO: 15)
**Figure 3** shows the effect of peptide nucleic acid (PNA) addition on a DNA amplification with outer primers for the amplification of both VHH-CH2-CH3 and VH-CH1-CH2-CH3 encoding fragments.
**Figure 4** shows the inhibition dependence on PNA concentration in a DNA amplification with outer primers for the amplification of both VHH-CH2-CH3 and VH-CH1-CH2-CH3 encoding fragments. The ladder indicates that VHH band is approximately 600 bp and VH band is approximately 900 bp.
**Figure 5** shows the influence of template DNA concentration on DNA amplification inhibition of VH-CH1-CH2-CH3 encoding fragments.
**Figure 6** shows the sequence map of pASF103.

### Description of the sequence listing

The sequence listing provides at least the following sequences of Table 1.

**Table 1: List of sequences used for the current invention**

| SEQ ID NO: | Nucleotide | Description |
|---|---|---|
| | Sequence (5'-> 3') | |
| 1 | GCAAGACCTTCATCTGCAACGTAG | CH1-PNA-sense |
| 2 | TTGTCCACCTTGGTGCTGCT | CH1-PNA-antisense |
| 3 | GGCTGAGCTGGGTGGTCCTGG | Lib1 |
| 4 | GGCTGAGTTTGGTGGTCCTGG | Lib2 |
| 5 | GGTACGTGCTGTTGAACTGTTCC | Lib3 |
| 6 | TCCACCAAGGCCCCATCGGTCTATCCTCTGACTGCTAGATGCGGGG ACACGCCTGGCTCCACAGTGGCCTTCGGCTGCCTGGTCTGGGGCTA CATCCCTGAGCCGGTGACGGTGACTTGGAACTCAGGCGCCCTGTCC AGCGGCGTCCACACCTTCCCATCAGTCTTCATGTCCTCGGGGCTCTA CTCCCTCAGCAGCTTGGTGACACTGCCCACAAGCAGCTCAACCGGC AAGACCTTCATCTGCAACGTAGCCCACCCGGCCAGCAGCACCAAGG TGGACAAGCGTGTGG | CH1 region of *Lama glama* IgG1b, clone D10 |
| 7 | TCCACCAAGGCCCCATCGGTCTATCCTCTGACTGCTAGATGCGGGG ACACGCCTGGCTCCACAGTGGCCTTCGGCTGCCTGGTCTGGGGCTA CATCCCTGAGCCGGTGACGGTGACTTGGAACTCAGGCGCCCTGTCC AGCGGCGTCCACACCTTCCCATCAGTCTTCATGTCCTCGGGGCTCTA CTCCCTCAGCAGCTTGGTGACACTGCCCACAAGCAGCTCAACCGGC AAGACCTTCATCTGCAACGTAGCCCACCCGGCCAGCAGCACCAAGG TGGACAAGCGTGTGG | CH1 region of *Lama glama* IgG1b, AF305955 |
| 8 | TCCACCAAGGCCCCATCGGTCTATCCTCTGACTGCTAGATGCGGGG ACACGCCTGGCTCCACAGTGGCCTTCGGCTGCCTAGTCTGGGGCTA TATCCCTGAGCCGGTGACGGTGACGTGGAACTCGGGCGCCCTGTC CAGCGGCGTCCACACCTTCCCATCAGTCTTCATGTCCTCGGGGCTCT ACACCCTCAGCAGCTTGGTGACCATGCCCGCCAGCAGCTCGACCGG CAAGACCTTCATCTGCAACGTAGCCCACCCGGCCAGCAGCACCAAG GTGGACAAGCGTGTGG | CH1 region of *Vicugna pacos* IgG1a, AM773729 |
| 9 | TCCACCAAGGCCCCATCGGTCTATCCTCTGACTGCTAGATGCGGGG ACACGCCTGGCTCCACGGTGGCCTTCGGCTGCCTGGTCTGGGGCTA CATCCCTGAGCCGGTGACGGTGACCTGGAACTCGGGCGCCCTGTCC AGCGGCATCCACACCTTCCCATCTGTTCTCATGTCCTCGGGGCTCTA CTCACTCAGCAGCTTAGTGACTCTGCCCGCCAGCAGCTCGTCCGGC AAGAACTTCATCTGCAACGTAGCCCACCCGGCCAGCAGCACTAAGG TGGACAAGCGTGTGG | CH1 region of *camelus dromedarius* IgG1a, HE653043 |
| 10 | TCCACCAAGGCCCCATCGGTCTATCCTCTGACTGCTAGATGGGGGG ACACGCCTGGCTCCACGGTGGCCTTCGGCTGCCTGGTGTGGGGCTA CATCCCTGAGCCAGTGACAGTGACCTGGAACTCGGGTGCCCTGTCC AGCGGCGTCCACACCTTCCCGTCAGTCTTCATGTCCTCGGGGCTCTA CACCCTCAGCAGCTTAGTGACTCTGCCCACCAGCAGCTCGACCGGC AAGACCTTCTTCTGCAACGTAGCCCACCCGGCCAGCAGCACCAAGG TGGACAAGCGTGTGG | CH1 region of *camelus dromedarius* IgG1b, HE653081 |
| 11 | TCCACCAAGGCCCCATCGGTCTATCCTCTGACTGCTAGATCCGGGG ATACGCCTGGCTCCACGGTGGCCTTCGGCTGCCTGGTCTGGGGCTA CATCCCTGAGCCGGTGACGGTGACCTGGAATTCAGGCGCTGTGTCC AGCGGCATCCACACCTTCCCATCTGTTCTCATGTCCTTGGGGCTCTA CTCACTCAGCAGCTTAGTGACTCTGCCCACCAGCACCTCGACCGGA AAGACCTTCATCTCCAACGTAGCCCACCCAGCCAGCAGCACCAAGG TGGACAAGAGTGTGG | CH1 region of *camelus dromedarius* IgG2a, AJ1311945 |
| 12 | TCCACCAAGGCCCCATCGGTCTATCCTCTGACTGCTAGATGCGGGG ACACGCCTGGCTCCACGGTGGCCTTCGGCTGCCTGGTCTGGGGCTA CATCCCTGAGCCGGTGACGGTGACCTGGAACTCGGGCGCCCTGTCC AGCGGCATCCACACCTTCCCATCTGTTCTCATGTCCTCGGGGCTCTA CTCACTCAGCAGCTTAGTGACTCTGCCCGCCAGCAGCTCGTCCGGC AAGAACTTCATCTGCAACGTAGCCCACCCGGCCAGCAGCACTAAGG TGGACAAGCGTGTGG | CH1 region of *Camelus bacterianus* IgG1a, HE653055 |
| 13 | TCCACCAAGGCCCCATCGGTCTATCCTCTGACTGCTAGATGCGGGG ACACGCCTGGCTCCACGGTGGCCTTCGGCTGCCTGGTCTGGGGCTA CATCCCTGAGCCGGTGACGGTGACCTGGAACTCGGGTGCCCTGTCC AGCGGCGTCCACACCTTCCCGTCAGTCTTCATGTCCTCGGGGCTCTA CACCCTCAGCAGCTTAGTGACTCTGCCCACCAGCAGCTCGACCGGC AAGACCTTCTTCTGCAACGTAGCCCACCCGGCCAGCAGCACCAAGG TGGACAAGCGTGTGG | CH1 region of *Camelus bacterianus* IgG1b, HE653094 |
| 14 | TCCACCAAGGCCCCATCGGTCTATCCTCCGACTGCTGGATGCTGGG ACATGCCTGACTCCACGGTGGCCTTCGGCTGCCTGGTCTGGGGCTA CATCCCTGAGCCGGTGACGGTGACCTGGAACTCGGGCGCCCTGTCC AGCGGCATCCACACCTTCCCGTCCATTCTCATGTCCTTGGGTCTCTA CTCCCTCAGCAGCTTGGCGACCATGCCCGCCAGCAGCTCGGCCGGC AAGACCTTCATCTGCAACGTAGCCCACCCGGCCAGCAGCACCAAGG CGGACAAGCGTGTGG | CH1 region of *Camelus bacterianus,* XR_004318543 |
| 15 | TCCACCAAGGCCCCATCGGTCTATCCTCCGACTGCTGGATGCGGGG ACGCTCCTGGCTCCACGGTGCCCTTCGGCTGCCTGGTCTGGGGCTA CATCCCTGAGCCGGTGACGGTGACCTGGAACTCGGGCGCCCTGTCC AGCGGCATCCACACCTTCCCGTCCATTCTCATGTCCTCGGGGCTCTA CTCCCTCAGCAGCTTGGCGACCATGCCCGCCAGCAGCTCGGCCGGC AAGACCTTCATCTGCAACGTAGCCCACCCGGTCAGCAGCACCAAGG CGGACAAGCATGTGG | CH1 region of *Camelus bacterianus,* XR_004313296 |
| 16 | **TGATGCTCTTCCGCT**GAGGAGACGGTGACCTGGGT | Library Forward primer 5- Sapl nested |
| 17 | **CTTGGCTCTTCTGTG**CAGCTGCAGGAGTCTGGGGGAGG | Library Reverse primer 7 - Sapl nested |
| 18 | TTTACGGTTCCTGGCCTTTTGCTGGCCTTTTGCTCACATGTTCTTTCC TGCGTTATCCCCTGATTCTGTGGATAACCGTATTACCGCCTTTGAGT GAGCTGATACCGCTCGCCGCAGCCGAACGACCGAGCGCAGCGAGT CAGTGAGCGAGGAAGCGGAGGAGCGCCCAATACGCAAACCGCCTC TCCCCGCGCGTTGGCCGATTCATTAATGCAGCTGGCACGACAGGTT TCCCGACTGGAAAGCGGGCAGTGAGCGCAACGCAATTAATGTGAG TTAGCTCACTCATTAGGCACCCCAGGCTTTACACTTTATGCTTCCGG CTCGTATGTTGTGTGGAATTGTGAGCGGATAACAATTTCACAAGCT TAAGGAGACAGTACATATGAAATACCTATTGCCTACGGCAGCCGCT GGATTGTTATTACTCGCGGCCCAGCCGGCCATGGCCGACGTGCGA AGAGCGAGGCATGATCCGCTCTTCGAGTTGATCGTACGTAGCTCGA GGAGCCGATCAGCTCTTCCAGCGGCCGCAGACTACAAAGACCATG ACGGTGATTATAAAGATCATGACATCGATTACAAGGATGACGATGA CAAGGCCGCATAGACTGTTGAAAGTTGTTTAGCAAAACCTCATACA GAAAATTCATTTACTAACGTCTGGAAAGACGACAAAACTTTAGATC GTTACGCTAACTATGAGGGCTGTCTGTGGAATGCTACAGGCGTTGT GGTTTGTACTGGTGACGAAACTCAGTGTTACGGTACATGGGTTCCT ATTGGGCTTGCTATCCCTGAAAATGAGGGTGGTGGCTCTGAGGGT GGCGGTTCTGAGGGTGGCGGTTCTGAGGGTGGCGGTACTAAACCT CCTGAGTACGGTGATACACCTATTCCGGGCTATACTTATATCAACCC TCTCGACGGCACTTATCCGCCTGGTACTGAGCAAAACCCCGCTAAT CCTAATCCTTCTCTTGAGGAGTCTCAGCCTCTTAATACTTTCATGTTT CAGAATAATAGGTTCCGAAATAGGCAGGGTGCATTAACTGTTTATA CGGGCACTGTTACTCAAGGCACTGACCCCGTTAAAACTTATTACCA GTACACTCCTGTATCATCAAAAGCCATGTATGACGCTTACTGGAAC GGTAAATTCAGAGACTGCGCTTTCCATTCTGGCTTTAATGAGGATC CATTCGTTTGTGAATATCAAGGCCAATCGTCTGACCTGCCTCAACCT CCTGTCAATGCTGGCGGCGGCTCTGGTGGTGGTTCTGGTGGCGGC TCTGAGGGTGGCGGCTCTGAGGGTGGCGGTTCTGAGGGTGGCGG CTCTGAGGGTGGCGGTTCCGGTGGCGGCTCCGGTTCCGGTGATTTT GATTATGAAAAAATGGCAAACGCTAATAAGGGGGCTATGACCGAA AATGCCGATGAAAACGCGCTACAGTCTGACGCTAAAGGCAAACTT GATTCTGTCGCTACTGATTACGGTGCTGCTATCGATGGTTTCATTGG TGACGTTTCCGGCCTTGCTAATGGTAATGGTGCTACTGGTGATTTTG CTGGCTCTAATTCCCAAATGGCTCAAGTCGGTGACGGTGATAATTC ACCTTTAATGAATAATTTCCGTCAATATTTACCTTCTTTGCCTCAGTC GGTTGAATGTCGCCCTTATGTCTTTGGCGCTGGTAAACCATATGAA TTTTCTATTGATTGTGACAAAATAAACTTATTCCGTGGTGTCTTTGC GTTTCTTTTATATGTTGCCACCTTTATGTATGTATTTTCGACGTTTGC TAACATACTGCGTAATAAGGAGTCTTAATAAGAATTCACTGGCCGT CGTTTTACAACGTCGTGACTGGGAAAACCCTGGCGTTACCCAACTT AATCGCCTTGCAGCACATCCCCCTTTCGCCAGCTGGCGTAATAGCG AAGAGGCCCGCACCGATCGCCCTTCCCAACAGTTGCGCAGCCTGAA TGGCGAATGGCGCCTGATGCGGTATTTTCTCCTTACGCATCTGTGC GGTATTTCACACCGCATACGTCAAAGCAACCATAGTACGCGCCCTG TAGCGGCGCATTAAGCGCGGCGGGTGTGGTGGTTACGCGCAGCGT GACCGCTACACTTGCCAGCGCCTTAGCGCCCGCTCCTTTCGCTTTCT TCCCTTCCTTTCTCGCCACGTTCGCCGGCTTTCCCCGTCAAGCTCTAA ATCGGGGGCTCCCTTTAGGGTTCCGATTTAGTGCTTTACGGCACCTC GACCCCAAAAAACTTGATTTGGGTGATGGTTCACGTAGTGGGCCAT CGCCCTGATAGACGGTTTTTCGCCCTTTGACGTTGGAGTCCACGTTC TTTAATAGTGGACTCTTGTTCCAAACTGGAACAACACTCAACTCTAT CTCGGGCTATTCTTTTGATTTATAAGGGATTTTGCCGATTTCGGTCT ATTGGTTAAAAAATGAGCTGATTTAACAAAAATTTAACGCGAATTTT AACAAAATATTAACGTTTACAATTTTATGGTGCACTCTCAGTACAAT CTGCTCTGATGCCGCATAGTTAAGCCAGCCCCGACACCCGCCAACA CCCGCTGACGCGCCCTGACGGGCTTGTCTGCTCCCGGCATCCACTT ACAGACAAGCTGTGACCGTCTCCGGGAGCTGCATGTGTCAGAGGT TTTCACCGTCATCACCGAAACGCGCGAGACGAAAGGGCCTCGTGAT ACGCCTATTTTTATAGGTTAATGTCATGATAATAATGGTTTCTTAGA CGTCAGGTGGCACTTTTCGGGGAAATGTGCGCGGAACCCCTATTTG TTTATTTTTCTAAATACATTCAAATATGTATCCGCTCATGAGACAATA ACCCTGATAAATGCTTCAATAATATTGAAAAAGGAAGAGTATGAGT ATTCAACATTTCCGTGTCGCCCTTATTCCCTTTTTTGCGGCATTTTGC CTTCCTGTTTTTGCTCACCCAGAAACGCTGGTGAAAGTAAAAGATG CTGAAGATCAGTTGGGTGCACGAGTGGGTTACATCGAACTGGATC TCAACAGCGGTAAGATCCTTGAGAGTTTTCGCCCCGAAGAACGTTT TCCAATGATGAGCACTTTTAAAGTTCTGCTATGTGGCGCGGTATTAT CCCGTATTGACGCCGGGCAAGAGCAACTCGGTCGCCGCATACACTA TTCTCAGAATGACTTGGTTGAGTACTCACCAGTCACAGAAAAGCAT CTTACGGATGGCATGACAGTAAGAGAATTATGCAGTGCTGCCATAA CCATGAGTGATAACACTGCGGCCAACTTACTTCTGACAACGATCGG AGGACCGAAGGAGCTAACCGCTTTTTTGCACAACATGGGGGATCA TGTAACTCGCCTTGATCGTTGGGAACCGGAGCTGAATGAAGCCATA CCAAACGACGAGCGTGACACCACGATGCCTGTAGCAATGGCAACA ACGTTGCGCAAACTATTAACTGGCGAACTACTTACTCTAGCTTCCCG GCAACAATTAATAGACTGGATGGAGGCGGATAAAGTTGCAGGACC ACTTCTGCGCTCGGCCCTTCCGGCTGGCTGGTTTATTGCTGATAAAT CTGGAGCCGGTGAGCGTGGGTCTCGCGGTATCATTGCAGCACTGG GGCCAGATGGTAAGCCCTCCCGTATCGTAGTTATCTACACGACGGG GAGTCAGGCAACTATGGATGAACGAAATAGACAGATCGCTGAGAT AGGTGCCTCACTGATTAAGCATTGGTAACTGTCAGACCAAGTTTAC TCATATATACTTTAGATTGATTTAAAACTTCATTTTTAATTTAAAAGG ATCTAGGTGAAGATCCTTTTTGATAATCTCATGACCAAAATCCCTTA ACGTGAGTTTTCGTTCCACTGAGCGTCAGACCCCGTAGAAAAGATC AAAGGATCTTCTTGAGATCCTTTTTTTCTGCGCGTAATCTGCTGCTT GCAAACAAAAAAACCACCGCTACCAGCGGTGGTTTGTTTGCCGGAT CAAGAGCTACCAACTCTTTTTCCGAAGGTAACTGGCTTCAGCAGAG CGCAGATACCAAATACTGTTCTTCTAGTGTAGCCGTAGTTAGGCCA CTACTTCAAGAACTCTGTAGCACCGCCTACATACCTCGCTCTGCTAA TCCTGTTACCAGTGGCTGCTGCCAGTGGCGATAAGTCGTGTCTTAC CGGGTTGGACTCAAGACGATAGTTACCGGATAAGGCGCAGCGGTC GGGCTGAACGGGGGGTTCGTGCACACAGCCCAGCTTGGAGCGAAC GACCTACACCGAACTGAGATACCTACAGCGTGAGCTATGAGAAAG CGCCACGCTTCCCGAAGGGAGAAAGGCGGACAGGTATCCGGTAAG CGGCAGGGTCGGAACAGGAGAGCGCACGAGGGAGCTTCCAGGGG GAAACGCCTGGTATCTTTATAGTCCTGTCGGGTTTCGCCACCTCTGA CTTGAGCGTCGATTTTTGTGATGCTCGTCAGGGGGGCGGAGCCTAT GGAAAAACGCCAGCAACGCGGCCTT | pASF103 |

### Detailed description of the invention

Reference to any prior art in this specification is not, and should not be taken as, an acknowledgment or any form of suggestion that this prior art forms part of the common general knowledge in any country.

All documents cited in the present specification are hereby incorporated by reference in their entirety. Unless otherwise defined, all terms used in disclosing the invention, including technical and scientific terms, have the meaning as commonly understood by one of ordinary skill in the art to which this invention belongs.

The present invention will be described with respect to particular embodiments but the invention is not limited thereto but only by the claims.

The present invention relates to an improved method for constructing a VHH antibody library. A "VHH antibody library" as used herein refers to a collection of different VHH sequences introduced into a vector such as a phagemid vector using common recombinant DNA technologies. A VHH antibody library may be used to screen and select for VHH binding to a certain antigen. One such an example is by using a VHH antibody library in a phagemid vector to select for VHH binding to a certain antigen by phage display, but other means of displaying and selecting VHH may be used, such as yeast display. Improving the construction of a VHH antibody library may increase its variability (i.e. the VHH sequence variation captured by the library), hereby improving the chances of selecting higher quantities and/or improved chances of selecting VHH antibodies having certain desired characteristics. The current invention improves the generation of a VHH antibody library by using a novel method of amplifying the VHH-CH2 amplicon from a cDNA preparation derived from an RNA extract from peripheral blood mononuclear cells of a *Camelidae* species by DNA amplification of the VHH-CH2 encoding regions present in the cDNA preparation, whilst at the same time blocking DNA amplification of VH-CH1-CH2 encoding region from a conventional antibody present in the cDNA preparation, hereby omitting the need to separate through gel filtration (or other method) the VHH-CH2 construct from the VH-CH1-CH2 constructs. In one embodiment, DNA amplification of VHH-CH2 encoding regions is
- initiated by a primer pair comprising a forward and a reverse primer, where the forward primer hybridizes to and/or upstream of the FR1 domain of the VHH-CH2 and VH-CH1-CH2 encoding regions and where the reverse primer hybridizes to and/or downstream of the CH2 domain of the VHH-CH2 and VH-CH1-CH2 encoding regions, thereby providing VHH-CH2 encoding amplicons; and
- wherein in the method of the present invention DNA amplification of VH-CH1-CH2 encoding regions is blocked by at least one blocking oligonucleotide.

In some embodiments according to the invention, the *Camelidae-species* has been immunized prior to obtaining peripheral blood mononuclear cells therefrom.

In a preferred embodiment the blocking oligonucleotide hybridizes to the CH1 domain of the VH-CH1-CH2 encoding region. In a more preferred embodiment the blocking oligonucleotide hybridizes to the CH1 domain comprising a polynucleotide sequence according to any one of SEQ ID NO: 6 to 15, or a polynucleotide sequence with, and with increasing preference, 90%, 91%, 92%, 93%, 94%, 95%, 96%, 97%, 98%, 99% or 100% identity thereto. The blocking oligonucleotide may hybridize to either the sense or the anti-sense strand of said CH1 domain.

In a more specific embodiment, the method of constructing a VHH antibody library as provided herein is such that the VH-CH1-CH2 encoding region is blocked by two or more blocking oligonucleotides as described herein, wherein at least one blocking oligonucleotide hybridizes to the sense strand of the CH1 domain and/or at least one blocking oligonucleotide hybridizes to the anti-sense strand of the CH1 domain. In an embodiment where two or more blocking oligonucleotides as described herein are used, it is preferred that at least one blocking oligonucleotides hybridizes to the sense strand and at least one blocking oligonucleotides hybridizes to the anti-sense strand.

In some embodiments, the method of constructing a VHH antibody library as provided herein is such that the blocking oligonucleotide is a non-priming oligonucleotide. A non-priming oligonucleotide, (also referred to as a non-extendable oligonucleotide) as used herein, is an oligonucleotide that has been chemically modified or designed to prevent DNA or RNA polymerases from extending the oligonucleotide. The mechanism of action typically involves modifications at the 3'-end or along the backbone of the oligonucleotide, rendering it incapable of serving as a primer for the DNA polymerase. This ensures that the polymerase cannot incorporate additional nucleotides or proceed with strand synthesis, thereby halting amplification at the intended site. Modifications to non-priming oligonucleotides can broadly be categorized as 3'-end modifications or backbone modifications. At a broad level, modifications to the 3'-end often involve substitution of the hydroxyl (-OH) group at the 3'-end with a non-reactive functional group such as a hydrogen (-H), phosphate group (-PO4), or other chemically inert groups. Additionally, bulky groups or fluorophores may be added at the 3'-end to physically block polymerase access. Backbone modifications, on the other hand, can involve alterations such as the incorporation of phosphorothioate linkages, which are resistant to nuclease activity and inhibit extension under certain conditions. Another example of backbone modification includes the replacement of the ribose or deoxyribose sugar backbone with synthetic analogues that reduce or eliminate polymerase recognition. More specifically, certain advanced modifications exemplify non-priming oligonucleotide design. Locked Nucleic Acids (LNA) are one such modification where the ribose ring of the oligonucleotide is chemically modified to contain a methylene bridge between the 2' oxygen and the 4' carbon. This structural alteration increases binding affinity to complementary sequences while preventing polymerase extension due to steric hindrance. Similarly, Peptide Nucleic Acids (PNA) replace the sugar-phosphate backbone of the oligonucleotide with a peptide-like backbone. This modification maintains the ability to hybridize to complementary DNA or RNA sequences while ensuring that the absence of a reactive 3'-end and the unique backbone structure prevent extension by DNA polymerases.

In some embodiments, the method of constructing a VHH antibody library as provided herein is such that the blocking oligonucleotide is a LNA or a PNA.

In a preferred embodiment, where the blocking oligonucleotide is a LNA, the DNA amplification step of the method of the present invention may be performed using a polymerase lacking 5'-3' and 3'-5' exonuclease activity.

In a more preferred embodiment, the method of constructing a VHH antibody library as provided herein is such that the blocking oligonucleotide is a PNA. In an embodiment where the blocking oligonucleotide is a PNA, a polymerase having 5'-3' and/or 3'-5' exonuclease activity may be used to achieve the same effect of blocking the DNA amplification of the VH-CH1-CH2 domain.

The blocking oligonucleotide according to the invention may have different lengths whereby a longer blocking oligonucleotide will hybridize at a higher temperature compared to a shorter blocking oligonucleotide, all other factors being equal. It is said that a longer blocking oligonucleotide may have a higher melting temperature, the skilled person will understand that the melting temperature will further depend on the GC content, the length, as well as the local GC content on the 5'and/or 3' end of the blocking oligonucleotide and other components such as salt concentrations. The skilled person will know how to optimize the melting temperature as to allow successful and specific annealing during DNA amplification. In one embodiment, the blocking oligonucleotide has a length of between 10 and 100 nucleotides. More preferably between 10 and 50 nucleotides, even more preferably between 15 and 30 nucleotides. In an even more preferred embodiment, the blocking oligonucleotide has a length of between 18 and 25 nucleotides. In a more preferred embodiment said blocking oligonucleotide is a PNA. In a more preferred embodiment, the at least one blocking oligonucleotide comprises a nucleotide sequence chosen from SEQ ID NOs: 1 or 2. In an even more preferred embodiment the at least one blocking oligonucleotide comprises a nucleotide sequence chosen from SEQ ID NOs: 1 or 2 and the at least one blocking oligonucleotide is a PNA. In some embodiments, the blocking oligonucleotide as described herein is present in a concentration of from 0.25 µM to 5 µM or higher, more preferably of from 0.625 µM to 2.5 µM, most preferably 1.25 µM or 2.5 µM, and where in a preferred embodiment the blocking oligonucleotide is a PNA. Importantly, the efficiency of the blocking is dependent on the amount of template cDNA used in the DNA amplification step of the current invention. In one embodiment, from 0.0001 µg to 100 µg of template cDNA is used, more preferably of from 0.0001 µg to 10 µg of template DNA, most preferably of from 0.001 µg to 1 µg of template DNA is used in the DNA amplification step.

The present invention relates to DNA amplification of the VHH-CH2 encoding regions present in the cDNA preparation whilst simultaneously blocking the amplification of VH-CH1-CH2 regions. In a preferred embodiment said amplification is performed by a polymerase chain reaction (PCR). In some specific embodiments, the forward primer comprises a nucleotide sequence according to SEQ ID NOs: 3 and/or 4 and where the reverse primer comprises a nucleotide sequence according to SEQ ID NO: 5. In a preferred embodiment, the DNA amplification of the VHH-CH2 encoding regions present in the cDNA preparation is performed with both a forward primer comprising the polynucleotide sequence SEQ ID NO: 3 and a second forward primer comprising the polynucleotide sequence according to SEQ ID NO:4 and a reverse primer comprising the polynucleotide sequence according to SEQ ID NO: 5. In a subsequent step, the method of constructing a VHH antibody library as provided herein comprises an additional DNA amplification step whereby the VHH antibody encoding regions present in the VHH-CH2 encoding amplicons are amplified, thereby providing VHH antibody encoding amplicons. In one embodiment, the primers used for the additional DNA amplification step whereby the VHH antibody encoding regions present in the VHH-CH2 encoding amplicons are amplified, optionally comprise additional nucleotides at the 5'-terminus to facilitate further cloning steps. Such additional nucleotides may for instance comprise restriction enzyme digestion enzyme recognition sites, such as but not limited to Sapl restriction sites. In an alternative embodiment the optional additional nucleotides at the 5'-terminus have homology to the cloning sites of a chosen vector to facilitate cloning by for instance Gibson assembly. The skilled person will know the various options to its disposal for cloning a DNA fragment into a vector, preferably a phagemid vector. In a specific embodiment the VHH antibody encoding region may be amplified using the forward and reverse primer pair according to SEQ ID NO: 16 and 17. In some embodiments, said VHH antibody encoding region may be further introduced into a suitable vector, preferably a vector suitable for displaying said VHH antibody library, preferably a phagemid vector.

Therefore, the current invention further relates to a method of obtaining one or more VHH binding to an antigen, comprising the steps of (i) providing the VHH library according to the current invention; (ii) expressing said VHH antibody library; (iii) screening said VHH antibody library for one or more VHH binding to said antigen; (iv) isolating said one or more VHH; and whereby the *Camelidae* species was immunized with said antigen prior to obtaining peripheral blood mononuclear cells therefrom; and where optionally the screening of said VHH antibody library for VHH antibody binding to said antigen is performed using phage display.

The present invention further relates to a kit of parts comprising: a primer pair suitable to amplify a VHH-CH2 and VH-CH1-CH2 encoding regions in a cDNA preparation derived from an RNA extract from peripheral blood mononuclear cells of a *Camelidae* species; the primer pair comprising a forward and a reverse primer, where the forward primer has the ability to hybridize to and/or upstream of the FR1 domain of the VHH-CH2 and VH-CH1-CH2 encoding regions and where the reverse primer has the ability to hybridize to and/or downstream of the CH2 domain of the VHH-CH2 and VH-CH1-CH2 encoding regions as further described herein; and a blocking oligonucleotide that is adapted to block DNA amplification of the VH-CH1-CH2 encoding regions by the primer pair as further described herein.

### Definitions

As used herein, the terms "comprising" and "including" are inclusive or open-ended and do not exclude additional unrecited elements, compositional components, or method steps. Accordingly, the terms "comprising" and "including" encompass the more restrictive terms "consisting essentially of" and "consisting of".

Where an indefinite or definite article is used when referring to a singular noun e.g. "a" or "an", "the", this includes a plural of that noun unless something else is specifically stated.

Unless indicated otherwise, all methods, steps, techniques and manipulations that are not specifically described in detail can be performed and have been performed in a manner known per se, as will be clear to the skilled person.

"Blocking", as used herein, refers to the reduction or inhibition of nucleotide amplification, for example, during DNA amplification in the presence of a non-priming oligonucleotide. Blocking can be quantitatively defined as the measurable decrease in the amount of PCR product (amplicons) generated when the reaction is conducted with the blocking oligonucleotide compared to a reaction performed under identical conditions without the blocking oligonucleotide. The degree of blocking is calculated as the difference in the final quantity of non-target (blocked) amplified product between the two reactions. A typical experimental setup for determining blocking efficiency involves performing DNA amplification of a target sequence using a specific set of primers in the presence and absence of the blocking oligonucleotide for a fixed number of cycles under optimized reaction conditions. Assessment of blocking can further be visually verified using a DNA gel electrophoresis where the presence of the blocking oligonucleotide results in a significant reduction or absence of amplified product in the blocked reaction compared to the unblocked control (See Examples 3-5). In some embodiments, blocking amplification with a blocking oligonucleotide according to the invention results in at least a 50% reduction in the amount of non-target amplified DNA product, particularly VH-CH1-CH2 conventional antibody sequences, compared to the unblocked reaction. In a further embodiment, at least 90%, such as at least 99%. In a particular embodiment, the amount of non-target amplified sequences is reduced at least 10² times in the presence compared to the absence of the blocking oligonucleotide, preferably at least 10³ times, more preferably at least 10⁴ times.

It is understood that the efficiency of blocking may depend on several factors, including concentration of the blocking oligonucleotide, the concentration of template DNA, annealing temperature of the non-priming oligo's and PCR conditions. The influence of these factors is discussed elsewhere herein. For example, influence of non-priming oligonucleotide concentration and template DNA concentration is discussed in Examples 4 and 5, respectively.

As used herein, the terms "polypeptide", "protein", "peptide", and "amino acid sequence" are used interchangeably, and refer to a polymeric form of amino acids of any length, which can include coded and non-coded amino acids, chemically or biochemically modified or derivatized amino acids, and polypeptides having modified peptide backbones. A VHH antibody is an example of a polypeptide.

The terms "nucleic acid" or "oligonucleotide" or "polynucleotide" may be used interchangeably and refer to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Polynucleotides may have any three-dimensional structure, and may perform any function, known or unknown. Non-limiting examples of polynucleotides include a gene, a gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, control regions, isolated RNA of any sequence, nucleic acid probes, blocking oligonucleotide, locked oligonucleotide, peptide nucleic acid, and primers. The nucleic acid molecule may be linear or circular.

As used herein, the term "vector" refers a circular, double-stranded DNA molecule, to which nucleic acid fragments, preferably the amplicons as described herein, may be inserted and cloned, i.e., propagated. Hence, a vector will typically contain one or more unique restriction sites, one or more inducible promoters, and may be capable of autonomous replication in a defined cell or vehicle organism such that the cloned sequence is reproducible. The skilled person will understand that for the present invention, the vector is a vector capable of replicating in bacterial cells. A vector as disclosed herein may be a phage display vector. A vector may also contain a selection marker, such as, e.g., an antibiotic resistance gene, to allow selection of recipient cells that contain the vector. Vectors may include, without limitation, plasmids, cosmids, phagemids, bacteriophage-derived vectors, PAC, BAC, etc., as appropriate. The plasmids may be plasmids intended for viral vector construction such as *inter alia* retroviral vectors, lentiviral vectors, adenoviral vectors, or adeno- associated viral vectors. Expression vectors are generally configured to allow for and/or effect the expression of nucleic acids or open reading frames introduced thereto in a desired expression system, e.g., in vitro, in a cell, organ and/or organism. For example, expression vectors may advantageously comprise suitable regulatory sequences.

As used herein, "cDNA preparation" or "complementary DNA preparation" refers to any complementary DNA (cDNA) prepared from any appropriate starting sample using any available method. In some embodiments, the cDNA may be prepared starting from blood sample. In some embodiments, the cDNA may be prepared starting from leukocytes. Preferably, the cDNA may be prepared starting from peripheral blood mononuclear cells. More preferably, the cDNA may be prepared starting from lymphocytes. In some embodiments, the cDNA may be prepared from B-cells. In preferred embodiments, the cDNA may be prepared starting from peripheral blood mononuclear cells, such as lymphocytes, of a *Camelidae* species.

As used herein, *"Camelidae"* refers to the phylogenetic family *Camelidae* and any members thereof. In particular, it refers to members of subfamily *Camelinae.* More particularly, it refers to any members of the tribe Lamini or Camelini. Non-limiting examples of members of *Camelidae* include *Camel, Llama, Guanaco, Alpaca, Dromedary, and Vicuña.*

The term "region" as used herein refers to any segment or portion of a nucleotide or peptide sequence, which may be defined by its sequence location, function, biochemical properties, or structural features. A region may be continuous or discontinuous, and its boundaries may be delineated based on context or application. An "encoding region" refers to a region of a nucleotide sequence that specifies the production of a functional RNA or protein product. Encoding regions may include open reading frames (ORFs), exons, or functional subsequences therein, such as codons for specific domains or motifs.

As used herein, "domain" refers to a particular entity in all its molecular forms, unless explicitly indicated otherwise. For example, CH1 domain may refer to the oligonucleotide or polypeptide sequence of the CH1 domain.

As used herein, the terms "upstream" and "downstream" refer to the generally known usage in the field. As will also be clear from the disclosures herein, the antibody domains are written and referred to as per the conventional use in the field. For example, in a heavy chain of a typical antibody, variable domain (e.g. VH) is located upstream of constant domains (e.g. CH1, CH2, etc.). Accordingly, in a heavy chain of a typical antibody, constant domains are located downstream of variable domain. To give an example, VH is located upstream of CH1, and CH2 is located downstream of both VH and CH1. In the context of a protein, an upstream domain, region or site would be a domain, region or site located towards the N-terminus of the protein, whereby a downstream domain, region of site would be a domain, region or site located towards the N-terminus of the protein. In the context of a polynucleotide (be it ssDNA, dsDNA, cDNA, RNA, mRNA or otherwise), an upstream domain, region or site would be a domain, region or site located towards the 5' end of the polynucleotide, whereby a downstream domain, region of site would be a domain, region or site located towards the 3' end of the polynucleotide.

"VHH antibodies", also known as VHHs, VHH antibody fragments, VHH domains, and nanobodies have typically been described as the antigen binding immunoglobulin domain of "heavy chain antibodies" (i.e. antibodies devoid of light chains or HCAbs). The term "VHH domain" has been chosen to distinguish these variable domains from the heavy chain variable domains that are present in conventional 4-chain antibodies (which are referred to herein as "VH domains") and from the light chain variable domains that are present in conventional 4-chain antibodies (which are referred to herein as "VL domains"). As used herein, VHH antibody refers to polypeptide of a heavy-chain antibody comprising at least a variable domain (i.e. VHH domain). From the context it is clear that VHH antibody refers to heavy chain antibodies, comprising for example VHH and optionally one or more constant domains, as well as any derivatives thereof, for example the VHH domain.

In some embodiments, VHH antibodies may arise from members of family *Camelidae.* In some embodiments, VHH antibodies may arise from immunized members of family *Camelidae.* In some embodiments, VHH antibodies may arise from non-immunized members of family *Camelidae.* In some embodiments, VHH antibodies do not comprise CH1 domain.

As used herein, "polymerase" refers to any enzyme capable of synthesizing long chains of polymers or nucleic acids. In particular, the term comprises enzymes having no exonuclease activity as well as enzymes having exonuclease activity, such as 5' - 3' exonuclease activity.

As used herein, the term "sequence identity" means that two polypeptide or polynucleotide sequences are identical (i.e. on an amino acid-by-amino acid, or on a nucleotide-by-nucleotide basis, respectively) over a window of comparison. The term " percentage of sequence identity" is calculated by comparing two optimally aligned sequences over the window of comparison, determining the number of positions at which the identical amino acid or nucleic acid base, whichever relevant, occurs in both sequences to yield the number of matched positions, dividing the number of matched positions by the total number of positions in the window of comparison (i.e. the window size), and multiplying the result by 100 to yield the percentage of sequence identity.

### Methods of preparing antibody libraries

Methods disclosed in the present invention can be used for preparing an antibody library, in particular a VHH antibody library.

Antibodies typically comprise two heavy chains (denoted as H) and two light chains (denoted as L), each comprising one variable domain (denoted as VH or VL) and one or more constant domains (denoted by CHn or CL, where n is 1 or more). For example, heavy-chain of a conventional antibody may be of the form VH-CH1-CH2-CH3 and light chain may be of the form VL-CL. Where a "hinge domain" or "hinge region" connects the CH1 and CH2 domain. Heavy chain only antibodies or HCAbs as disclosed herein refers to antibodies comprising heavy-chains whilst lacking light chains. The variable domains of HCAbs are denoted as VHH domains (sometimes referred to as nanobodies, VHH or VHH antibodies). HCAbs are further distinct from conventional antibodies in that the heavy-chain of HCAbs is shorter than conventional antibodies, as the CH1 domain is missing. a HCAbs may be of the form VHH-CH2-CH3. Where a hinge region connects the VHH and CH2 domain.

The variable domains of antibodies are further divided into framework regions (FR) and hypervariable regions or complementarity determining regions (CDR). There are typically four FR in VH or VHH, numbered successively starting from the N-terminus of the variable region (i.e. FR1 before FR2, FR3 and FR4) separating the 3 CDR domains numbered successively starting from the N-terminus of the variable region (i.e. CDR1, CDR2 and CDR3). VH or VHH may thus be subdivided into following regions from the N- to C-terminus: FR1-CDR1-FR2-CDR2-FR3-CDR3-FR4.

Production of specific antibodies relies on different methods of generating, purifying, selecting, or a combination thereof, particular antibodies of interest. One such methods relies on generating antibody libraries. Libraries can be generated from immunized donors, containing antibodies which are biased for recognising antigen of interest or fragments thereof (i.e. immune library). Libraries can also be generated from non-immunized donors (i.e. naïve libraries). Other types of libraries, such as synthetic or semisynthetic, alternatively rely on in silico design strategies.

Typically, immune libraries are generated by collecting blood samples from donors. Peripheral blood mononuclear cells from these blood samples are processed to extract total RNA. The RNA is thereafter used to generate a cDNA, for example using a reverse transcriptase PCR (RT-PCR). The cDNA is thereafter used as starting material for DNA amplification using standard primer sets which result in amplification of the target antibody sequences of interest.

In order to generate a VHH antibody library, the inventors have identified a method of using peripheral blood mononuclear cells sample from *Camelidae* species, wherein the sequence of the VHH domain of HCAbs is selectively amplified whilst the amplification of the VH domain of conventional antibodies is blocked. Specifically, the inventors have surprisingly identified a method comprising DNA amplification of VHH-CH2 encoding regions, thereby providing VHH-CH2 encoding amplicons, and wherein the DNA amplification of VH-CH1-CH2 encoding regions is blocked by at least one blocking oligonucleotide. Therefore, in some embodiments, the present invention provides a method of constructing a VHH antibody library, comprising the steps of providing a cDNA preparation derived from an RNA extract from peripheral blood mononuclear cells of a *Camelidae* species, and DNA amplification of VHH-CH2 encoding regions, thereby providing VHH-CH2 encoding amplicons, and wherein the DNA amplification of VH-CH1-CH2 encoding regions is blocked by at least one blocking oligonucleotide.

The method of the present invention therefore firstly relates to the step of DNA amplification initiated by a primer pair comprising a forward and a reverse primer. In a preferred embodiment of the invention, DNA amplification is accomplished through PCR, utilizing forward and reverse primers. The forward primer of the present invention hybridizes to the FR1 domain of the VHH-CH2 and VH-CH1-CH2 encoding regions. Alternatively, the forward primer according to the present invention hybridizes upstream to the FR1 domain of the VHH-CH2 and VH-CH1-CH2 encoding regions. As is clear from the context, the forward primer may hybridize to any conserved region in or upstream of the FR1 domain, such as a region in the FR1 domain just downstream of the start codon. In some embodiments the forward primer may hybridize to a region of the FR1 domain comprising the start codon, where in some embodiments such a primer binds both upstream of the FR1 domain as in the FR1 domain. Hence, the forward primer has the ability to hybridize to and/or upstream of, the FR1 domain of the VHH-CH2 and VH-CH1-CH2 encoding regions In some aspects of the invention 2 or more forward primers are used simultaneously to better capture the diversity of VHH domains comprising different FR1 domains.

The reverse primer according to the present invention hybridizes to the CH2 domain of the VHH-CH2 and VH-CH1-CH2 encoding regions. Alternatively, the reverse primer according to the present invention hybridizes downstream of the CH2 domain of the VHH-CH2 and VH-CH1-CH2 encoding regions. For example, the reverse primer may bind CH3 domain sequence. Alternatively, the reverse primer according to the present invention hybridizes to and downstream of the CH2 domain of the VHH-CH2 and VH-CH1-CH2 encoding regions. Hence the reverse primer has the ability to hybridize to and/or downstream of the CH2 domain of the VHH-CH2 and VH-CH1-CH2 encoding regions.

The method of the present invention further relates to the step of blocking the amplification of VH-CH1-CH2 encoding region. The amplification may be blocked using one or more blocking oligonucleotide.

As such, the present invention provides a method of constructing a VHH antibody library, comprising the steps of providing a cDNA preparation derived from an RNA extract from peripheral blood mononuclear cells of a Camelidae species; and performing DNA amplification of VHH-CH2 encoding regions present in the cDNA preparation, whilst blocking DNA amplification of VH-CH1-CH2 encoding regions present in the cDNA preparation, wherein DNA amplification of VHH-CH2 encoding regions is initiated by a primer pair comprising a forward and a reverse primer, where the forward primer hybridizes to or upstream of the FR1 domain of the VHH-CH2 and VH-CH1-CH2 encoding regions and where the reverse primer hybridizes to or downstream of the CH2 domain of the VHH-CH2 and VH-CH1-CH2 encoding regions, thereby providing VHH-CH2 encoding amplicons, and wherein DNA amplification of VH-CH1-CH2 encoding regions is blocked by at least one blocking oligonucleotide.

The present invention provides a method of constructing a VHH antibody library, such that the method includes a first step of immunizing a member of the *Camelidae* species prior to processing any sample therefrom. As such, in some embodiments, the present invention provides a method of constructing a VHH antibody library according to any of the embodiments herein, such that a member of *Camelidae* species has been immunized prior to obtaining peripheral blood mononuclear cells therefrom. In some embodiments, said member of *Camelidae* family has been immunized with a fungal target or with a suitable antigenic determinant based thereon or derived therefrom, such as an antigenic part, fragment, region, domain, loop, or other epitope thereof. In some embodiments, the antigenic determinant may be an extracellular part, region, domain, loop, or other extracellular epitope(s).

### Blocking oligonucleotides

The present invention relates to methods of constructing a VHH antibody library, comprising blocking amplification using blocking oligonucleotides. In some embodiments, the method according to the present invention comprises a blocking oligonucleotide which hybridizes to at least part of the CH1 domain of the VH-CH1-CH2 encoding region.

In some embodiments, the invention provides a method of library construction according to any of the embodiments herein, such that the blocking oligonucleotide hybridizes to the CH1 domain according to the polynucleotide of any of SEQ ID NO: 6 to 15. The present invention also provides a method of library construction according to any of the embodiments herein, such that the blocking oligonucleotide hybridizes to the CH1 domain comprising the polynucleotide according to SEQ ID NO: 6 to 15 or a polynucleotide with, and with increasing preference, 90%, 91%, 92%, 93%, 94%, 95%, 96%, 97%, 98%, 99% or 100% identity thereto and where the blocking oligonucleotide hybridizes to either the sense or the anti-sense strand of the CH1 domain as defined herein.

Wherein the blocking oligonucleotides may be a "locked nucleic acid" or "LNA" which are DNA/RNA sequences which typically comprise a methylene group between the 2'-O and 4'-C which "locks" the ribose ring. LNA nucleotides observe Watson-Crick base pairing rules like conventional primers but are at the same time resistant to exonuclease and endonuclease degradation, and show an improved specificity caused by higher thermal stability. LNA are therefore useful as blocking oligonucleotides. Other chemical modifications leading to DNA/RNA oligo's or primers resistant to exo- and/or endonuclease degradation may be used in the current invention.

In an alternative embodiment the blocking oligonucleotides may be a "Peptide Nucleic Acid" or "PNA". PNA are a synthetic polymer that mimic DNA or RNA. It has a peptide-like backbone and nucleobases that can form base pairs with complementary nucleic acids. PNA have high affinity and specificity for hybridizing with nucleic acids and is resistant to degradation such as exo- or endonuclease degradation. PNA are therefore useful as blocking oligonucleotides.

It being understood that the blocking oligonucleotide may be any type of oligonucleotide that can specifically hybridize to a target DNA region and that prevents a polymerase chain reaction to successfully amplify the DNA region to which the blocking oligonucleotide hybridizes.

The inventors surprisingly found that multiple blocking oligonucleotides can also be used for blocking the amplification of CH1 domain. Therefore, in some embodiments the invention provides a method of constructing a VHH antibody library according to any of the embodiments herein, such that the VH-CH1-CH2 encoding region is blocked by at least two blocking oligonucleotides, wherein at least one blocking oligonucleotide hybridizes to the sense strand of the CH1 domain and at least one blocking oligonucleotide hybridizes to the anti-sense strand of the CH1 domain. In some embodiments, the invention provides a method of constructing a VHH antibody library according to any of the embodiments herein, such that the VH-CH1-CH2 encoding region is blocked by more than two blocking oligonucleotides, wherein at least one blocking oligonucleotide hybridizes to the sense strand of the CH1 domain and at least one blocking oligonucleotide hybridizes to the anti-sense strand of the CH1 domain.

The inventors have also found that blocking oligonucleotide can be a locked nucleic acid.. Therefore, in some embodiments, the invention provides a method of constructing a VHH antibody library according to any of the embodiments herein, such that the VH-CH1-CH2 encoding region is blocked by a locked nucleic acid.

In a preferred embodiment, the invention provides a method of constructing a VHH antibody library according to any of the embodiments herein, such that the VH-CH1-CH2 encoding region is blocked by a peptide nucleic acid blocking oligonucleotide. The inventors have surprisingly found that blocking oligonucleotide can also be a peptide nucleic acid.

The present invention provides methods of constructing a VHH antibody library, comprising blocking amplification of VH-CH1-CH2 region using blocking oligonucleotides of various lengths. In some embodiments, the invention provides a method of constructing a VHH antibody library according to any of the embodiments herein, such that the blocking oligonucleotide has a length of between 10 and 100 nucleotides, or between 10 and 50 nucleotides, or between 15 and 30 nucleotides, or between 18 and 25 nucleotides, preferably between 10 and 50 nucleotides, more preferably between 15 and 30 nucleotides. In preferred embodiments of the present invention, the blocking oligonucleotide is between 18 and 25 nucleotides. The inventors have found that some flexibility is allowed in the length of the blocking oligonucleotide.

The present invention provides a method of constructing a VHH antibody library, comprising blocking amplification of VH-CH1-CH2 region using blocking oligonucleotides with specific sequences. As such, in some embodiments, the present invention provides a method of constructing a VHH antibody library according to any of the embodiments herein, such that at least one of the blocking oligonucleotide is chosen from any of the SEQ ID Nos: 1 to 2. The inventors have found that blocking oligonucleotides with said specific sequences are particularly suitable for constructing a VHH antibody library.

### Polymerases for library preparation

The present invention provides a method of constructing a VHH antibody library wherein DNA amplification is performed in the presence of suitable polymerase. In some embodiments, the present invention provides a method of constructing a VHH antibody library according to any of the embodiments herein, such that DNA amplification is performed using any polymerase enzyme. In some embodiments, the present invention provides a method of constructing a VHH antibody library according to any of the embodiments herein, such that DNA amplification is performed using a polymerase enzyme lacking 5' - 3' exonuclease activity. In some embodiments, the present invention provides a method of constructing a VHH antibody library according to any of the embodiments herein, such that DNA amplification is performed using a polymerase enzyme lacking 3' - 5' exonuclease activity. In some embodiments, the present invention provides a method of constructing a VHH antibody library according to any of the embodiments herein, such that DNA amplification is performed using a polymerase enzyme lacking both 5' - 3' and 3' - 5' exonuclease activity.

In some embodiments, the present invention provides a method of constructing a VHH antibody library wherein blocking oligonucleotide is a regular oligonucleotide, non-priming oligonucleotide, or locked nucleic acid (LNA) or a peptide nucleic acid (PNA) and wherein the polymerase enzyme is such that it lacks 5' - 3' and/or 3' - 5' exonuclease activity. In some embodiments, the blocking oligonucleotide is an LNA and the polymerase enzyme for DNA amplification lacks the 5' - 3' and/or 3' - 5' exonuclease activity.

In some embodiments, the present invention provides a method of constructing a VHH antibody library wherein blocking oligonucleotide is a peptide nucleic acid (PNA), and polymerase enzyme is any enzyme suitable for DNA amplification. As such, in some embodiments, the blocking oligonucleotide is an PNA and the polymerase enzyme for DNA amplification has 5' - 3' and/or 3' - 5' exonuclease activity.

### Processing of amplicons: vectors and expression

The present invention also provides a method of constructing a VHH antibody library wherein the method further comprises DNA amplification of VHH antibody encoding regions present in the VHH-CH2 encoding amplicons, thereby providing VHH antibody encoding amplicons.

The present invention provides a method of constructing a VHH antibody library wherein amplicons are further introduced into a suitable vector. As such, in some embodiments, the present invention provides a method of constructing a VHH antibody library according to any of the embodiments herein, such that any of the resulting amplicons are introduced into a suitable vector. In some embodiments, the vector is any expression vector, suitable for expressing in any host cells such as a yeast expression vector or an E. coli expression vector. In a more preferred embodiment, the vector is a vector suitable for use in a method for displaying and selecting VHH antibodies, such as a phage display or yeast display (i.e. the vectors are designed such to allow expression of the VHH antibody library in a manner suitable for phage and/or yeast display).

The present invention provides a method of constructing a VHH antibody library, such that VHH domain is expressed and isolated. As such, in some embodiments, the present invention provides a method of constructing a VHH antibody library according to any of the embodiments herein, such that VHH domain is expressed. In some embodiments, the present invention provides a method of constructing a VHH antibody library according to any of the embodiments herein, such that VHH domain is expressed and isolated.

Preparation of VHH antibodies of the present invention as discussed herein can involve biological methods, such as somatic hybridization; or genetic methods, such as the expression of a non-native DNA sequence encoding the desired antibody structure in a cell line or in an organism; chemical methods (e.g. by chemical coupling, genetic fusion, noncovalent associated or otherwise to one or more molecular entities, such as another antibody or fragment thereof); or combination thereof.

VHH antibodies can be subjected to sequence optimization and other optimization techniques, such as to improve physicochemical or other properties. In particular, humanized VHH antibodies may be single-domain antibodies in which at least one single amino acid residue is present (and in particular, at least one framework residue) that is and/or that contributes to humanization.

VHH antibodies of the present invention may be monospecific, bispecific, or multispecific. Multispecific indicates that such VHH antibody may be specific for different epitopes of one target antigen or polypeptide, or may contain antigen-binding domains specific for more than one target antigen or polypeptide. As used herein, "bispecific" refers to VHH antibody having the capacity to bind two distinct epitopes either on a single antigen or polypeptide, or on two different antigens or polypeptides.

### Kits for constructing a VHH antibody library

The present invention relates to kits comprising primers and blocking oligonucleotides as disclosed herein, for constructing a VHH antibody library. Therefore, some embodiments provide a kit for constructing a VHH antibody library for a *Camelidae* species, said kit comprising primers and blocking oligonucleotides.

In some embodiments, the present invention provides a kit of parts comprising a primer pair suitable to amplify a VHH-CH2 and VH-CH1-CH2 encoding regions. In some embodiments, the present invention provides a kit of parts comprising, together with any of the combinations of kit of parts herein, a primer pair suitable to amplify a VHH-CH2 and VH-CH1-CH2 encoding regions in a cDNA preparation derived from an RNA extract from peripheral blood mononuclear cells of a Camelidae species, the primer pair comprising a forward and a reverse primer, where the forward primer has the ability to hybridize to and/or upstream of, the FR1 domain of the VHH-CH2 and VH-CH1-CH2 encoding regions. In some embodiments, the present invention provides a kit of parts comprising, together with any of the combinations of kit of parts herein, a primer pair suitable to amplify a VHH-CH2 and VH-CH1-CH2 encoding regions in a cDNA preparation derived from an RNA extract from peripheral blood mononuclear cells of a Camelidae species, the primer pair comprising a forward and a reverse primer, where the forward primer has the ability to hybridize to and/or upstream of the FR1 domain of the VHH-CH2 and VH-CH1-CH2 encoding regions. In some embodiments, the present invention provides a kit of parts comprising, together with any of the combinations of kit of parts herein, a primer pair suitable to amplify a VHH-CH2 and VH-CH1-CH2 encoding regions in a cDNA preparation derived from an RNA extract from peripheral blood mononuclear cells of a Camelidae species, the primer pair comprising a forward and a reverse primer, where the reverse primer has the ability to hybridize to and/or downstream of the CH2 domain of the VHH-CH2 and VH-CH1-CH2 encoding regions. In some embodiments, the present invention provides a kit of parts comprising, together with any of the combinations of kit of parts herein, a primer pair suitable to amplify a VHH-CH2 and VH-CH1-CH2 encoding regions in a cDNA preparation derived from an RNA extract from peripheral blood mononuclear cells of a Camelidae species, the primer pair comprising a forward and a reverse primer, where the reverse primer has the ability to hybridize downstream of the CH2 domain of the VHH-CH2 and VH-CH1-CH2 encoding regions.

In some embodiments, the present invention provides a kit of parts comprising, together with any of the combinations of kit of parts herein, a primer pair suitable to amplify a VHH-CH2 and VH-CH1-CH2 encoding regions in a cDNA preparation derived from an RNA extract from peripheral blood mononuclear cells of a Camelidae species, the primer pair comprising a forward and a reverse primer. In a preferred embodiment the forward primer comprises a polynucleotide sequence according to SEQ ID NO: 3 or 4 and where the reverse primer comprises a polynucleotide sequence according to SEQ ID NO: 5. In an even more preferred embodiments, the present invention provides a kit of parts comprising, together with any of the combinations of kit of parts herein, a primer pair suitable to amplify a VHH-CH2 and VH-CH1-CH2 encoding regions in a cDNA preparation derived from an RNA extract from peripheral blood mononuclear cells of a Camelidae species, the primer pair comprising at least two forward primers and a reverse primer, where the at least two forward primers comprise a polynucleotide sequence according to SEQ ID NO: 3 or 4, more preferably a first forward primer comprises a polynucleotide sequence according to SEQ ID NO: 3 and a second a polynucleotide sequence according to SEQ ID NO: 4; and where the reverse primer comprises a polynucleotide sequence according to SEQ ID NO: 5.

In some embodiments, the present invention provides a kit of parts comprising, together with any of the combinations of kit of parts herein, a blocking oligonucleotide that is adapted to block DNA amplification of the VH-CH1-CH2 encoding regions by the primer pair. In some embodiments, the present invention provides a kit of parts comprising, together with any of the combinations of kit of parts herein, a blocking oligonucleotide which hybridizes to at least part of the CH1 domain of the VH-CH1-CH2 encoding region. In a more preferred embodiment, the present invention provides a kit of parts comprising, together with any of the combinations of kit of parts herein, a blocking oligonucleotide which hybridizes to CH1 domain comprising the polynucleotide sequence according to any one of SEQ ID NO: 6 to 15, or a polynucleotide with, and with increasing preference, 90%, 91%, 92%, 93%, 94%, 95%, 96%, 97%, 98%, 99% or 100% identity thereto. In some embodiments, the present invention provides a kit of parts comprising, together with any of the combinations of kit of parts herein, a blocking oligonucleotide which hybridizes to the sense strand of the CH1 domain. In some embodiments, the present invention provides a kit of parts comprising, together with any of the combinations of kit of parts herein, a blocking oligonucleotide which hybridizes to the anti-sense strand of the CH1 domain.

In a more preferred embodiment, the present invention provides a kit of parts comprising, together with any of the combinations of kit of parts herein, at least two blocking oligonucleotides. In an even more preferred embodiment, of the at least two blocking oligonucleotides at least one hybridizes to the anti-sense strand of the CH1 domain and at least one hybridizes to the sense strand of the CH1 domain.

In some embodiments, the present invention provides a kit of parts comprising, together with any of the combinations of kit of parts herein, a blocking oligonucleotide which is a locked nucleic acid. In some embodiments, the present invention provides a kit of parts comprising, together with any of the combinations of kit of parts herein, a blocking oligonucleotide which is a peptide nucleic acid.

In some embodiments, the present invention provides a kit of parts comprising, together with any of the combinations of kit of parts herein, at least one blocking oligonucleotide having a length of between 10 and 100 nucleotides, more preferably between 10 and 50 nucleotides, more preferably between 15 and 30 nucleotides, more preferably between 18 and 25 nucleotides, even more preferably between 10 and 50 nucleotides, even more preferably between 15 and 30 nucleotides. In an even more preferred embodiment, said blocking oligonucleotide is between 18 and 25 nucleotides.

In a preferred embodiment, the present invention provides a kit of parts comprising, together with any of the combinations of kit of parts herein, at least one blocking oligonucleotide comprising a polynucleotide sequence according to SEQ ID Nos: 1 or 2. In a more preferred embodiment the present invention provides a kit of parts comprising, together with any of the combinations of kit of parts herein, at least two blocking oligonucleotide and where the first blocking oligonucleotide comprises a polynucleotide sequence according to SEQ ID NO: 1, and at least a second blocking oligonucleotide comprising a polynucleotide sequence according to SEQ ID NO: 2

In some embodiments, the present invention provides a kit of parts comprising a primer pair suitable to amplify a VHH-CH2 and VH-CH1-CH2 encoding regions in a cDNA preparation derived from an RNA extract from peripheral blood mononuclear cells of a Camelidae species, the primer pair comprising a forward and a reverse primer, where the forward primer has the ability to hybridize to and/or upstream of the FR1 domain of the VHH-CH2 and VH-CH1-CH2 encoding regions and where the reverse primer has the ability to hybridize to and/or downstream of the CH2 domain of the VHH-CH2 and VH-CH1-CH2 encoding regions, as describe herein; and a blocking oligonucleotide that is adapted to block DNA amplification of the VH-CH1-CH2 encoding regions by the primer pair as described herein; and where the kit of parts further comprises a further primer pair suitable to amplify the VHH antibody sequence of the VHH-CH2 amplicon obtained by the method of the current invention using the parts of the kit of parts according to the invention and wherein the further primer pair allows the VHH antibody sequence to be amplified and optionally cloned into a suitable vector, preferably a phagemid vector; and as further described herein. In a specific embodiment the VHH antibody encoding region may be amplified using the forward and reverse primer pair according to SEQ ID NO: 16 and 17.

### Methods of obtaining one or more VHH binding to an antigen

The invention further relates to methods of obtaining one or more VHH binding to an antigen. In some embodiments, said method comprises the steps of providing the VHH antibody library according to the invention or the VHH antibody library obtained using the kit according to the invention, expressing said VHH antibody library, screening said VHH antibody library for one or more VHH binding to the antigen, and isolating said one more VHH antibody. As discussed elsewhere herein, the VHH antibody library may be constructed from peripheral blood mononuclear cells of a *Camelidae* species. Preferably, the *Camelidae* species was immunized with said antigen prior to obtaining peripheral blood mononuclear cells therefrom.

In one embodiment, the isolation step involves selecting individual VHH antibodies that exhibit specific binding to the antigen of interest. The VHH antibody library is typically screened using an antigen-binding assay, such as enzyme-linked immunosorbent assay (ELISA), to identify candidates with the desired binding properties. The isolation process may further include amplification and cloning of the VHH sequences corresponding to the selected candidates. In some embodiments, isolated VHH sequences are subjected to sequencing to confirm their identity and to ensure that they meet predefined selection criteria.

The screening step ensures that VHH antibodies with high specificity and affinity for the antigen are identified. In some embodiments, screening is performed using phage display, wherein the VHH antibody library is expressed on the surface of bacteriophages, and binding to the antigen is assessed by panning (Examples 7-8). The panning process involves incubating the phage-displayed library with the immobilized antigen, washing to remove non-specific binders, and eluting specifically bound phages. Repeated rounds of panning may be performed to enrich for high-affinity VHH candidates. In another embodiment, yeast or ribosome display systems may be employed for screening, allowing for the rapid selection of high-affinity binders. Alternatively, screening may be performed in a high-throughput format, such as using microarray-based methods, where the antigen is immobilized, and the binding properties of the VHH antibodies are analyzed simultaneously.

The present invention will now be illustrated by way of the following non-limiting Examples.

### Examples

### Example 1: Immunizations

Llamas were immunized according to standard protocols with 3 to 4 injections of a suitable inoculum or antigen mixed with, for example, Gerbu adjuvant P (GERBU Biotechnik GmbH) and injected subcutaneously. All llamas remained healthy throughout the immunization process and blood samples were collected before and after the immunization experiment.

### Example 2: cDNA preparation

To generate cDNA, peripheral blood mononuclear cells were prepared from blood samples of the immunized llamas of Example 1 using FICOLL PAQUE PLUS (Merck) according to the manufacturer's instructions. Total RNA was extracted from these cells and used as starting material for RT-PCR to generate corresponding cDNA using the SuperScript^{™} IV First-Strand Synthesis System (Thermo Fisher Scientific).

### Example 3: Inhibitory effects of PNAs on different llama derived VH DNA

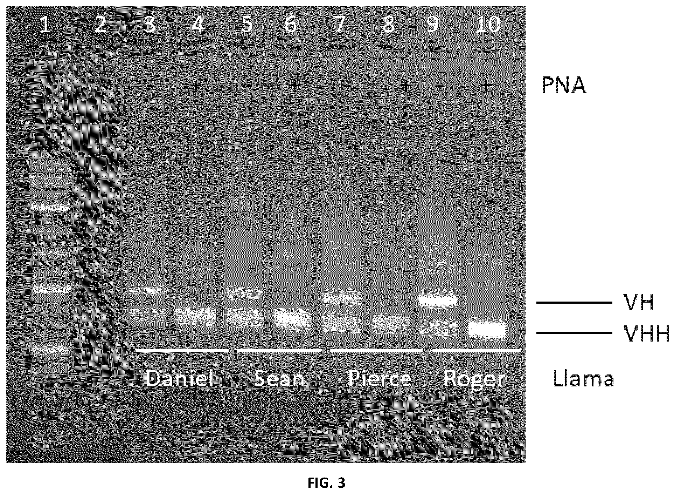

A set of two PNAs was designed (SEQ ID NO: 1 anneals to the sense strands, SEQ ID NO: 2 anneals to the antisense strand of the CH1 region) based on the *Lama glama* igG1b CH1 sequence of clone D10 (see Figure 2). However, other PNA oligo's may be designed based on the sequence of the sense and/or antisense strands of CH1 region and these PNA oligo's may be functional in different species as well (see for example the alignment of the CH1 region of different isotypes in Figure 2, showing that the here disclosed PNA will bind to any of SEQ ID NO: 6 to SEQ ID NO: 15). To determine if these PNAs generally inhibit the VH DNA amplification, the PNA mixture was tested on four llama derived cDNA samples obtained in Example 2, applying 25 cycles with Lib1 and Lib2 forward primer (SEQ ID NO: 3 and SEQ ID NO: 4) and Lib3 reverse primer (SEQ ID NO: 5), both at a concentration of 200nM, with the proofreading HIFI Taq polymerase according to manufacturer's instructions. The Lib1 and Lib2 anneal in the FR1 region just downstream of the start codon of the VH and VHH sequences and the Lib3 primer anneals in the CH2-CH3 region. As shown in Figure 3, only the samples without PNAs contain both the 700 bp VHH-CH2 amplicon and the 1000 bp VH-CH1-CH2 amplicon, while the reactions where PNAs were added at 2.5 µM only show the smaller 700 bp VHH band. Also, the intensity of the VHH-CH2 amplicon is enhanced if the amplification of the CH1-containing VH-CH1-CH2 is blocked by the PNAs (Figure 3).

### Example 4: PNA concentration

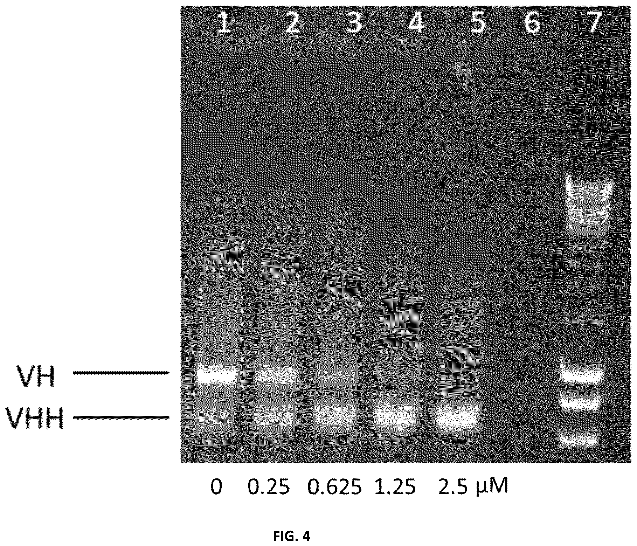

To evaluate the optimal concentration of PNAs to add to the DNA amplification mix, the same PCR as in Example 3 was performed with different PNA concentrations from 0 µM to 2.5 µM. Figure 4 shows that a 2.5 µM PNA concentration provides for a most optimal blocking of the VH-CH1-CH2 amplification.

### Example 5: Inhibition efficiency dependence on input DNA concentration

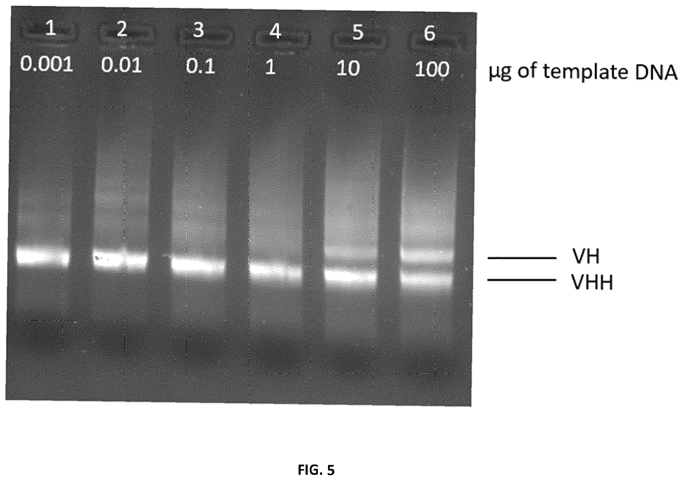

To evaluate the optimal concentration of template DNA in the DNA amplification mix, the same PCR as in Example 3 was performed with different template DNA concentrations from 0.001 µg to 100 µg, whilst keeping the concentration of PNAs constant at 2.5 µM. Figure 5 shows that a template DNA concentration of 1ng may already be sufficient for an efficient amplification of the VHH-CH2 region in the presence of PNAs at 2.5 µM.

### Example 6: nested PCR

The resulting VHH-CH2 amplicon was thereafter further amplified using a nested PCR, resulting in a VHH region lacking the CH2 region resulting from the PCR as performed in Example 3. The forward primer according to SEQ ID NO: 16 and the reverse primer according to SEQ ID NO: 17 were used for this amplification using standard PCR protocols using a proofreading polymerase. This PCR produced a VHH DNA amplicon flanked with Sapl restriction digestion sites for subsequent cloning.

### Example 7: cloning of DNA amplification product in phagemid vector

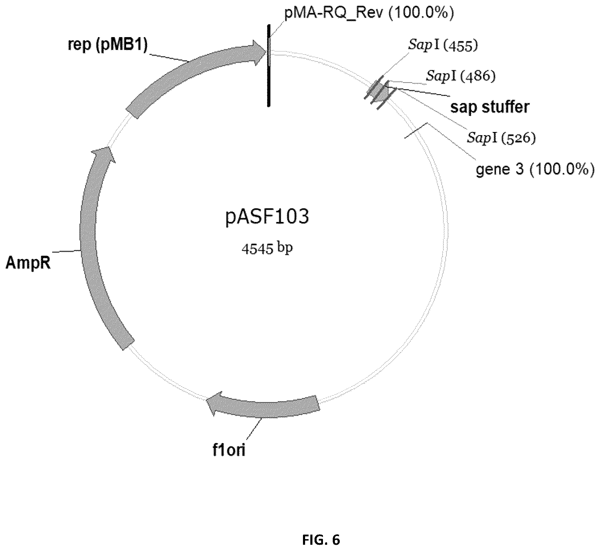

The resulting amplified VHH fragments of Example 6 were cloned into phagemid vector pASF103 (SEQ ID NO: 18 and Figure 6) into the corresponding Sapl sites, where pASF103 may be used as a phagemid vector in a standard phage display protocol. pASF103 is an expression vector derived from pUC119 which contains the lac promoter, a synthetic leader sequence, a multiple cloning site, a coliphage pIII protein encoding sequence, a resistance gene for ampicillin, and an f1 phage origin for single strand production. Phages were prepared according to standard methods (Phage Display of Peptides and Proteins: A Laboratory Manual; Brian K. Kay, Jill Winter, John McCafferty). Libraries with a clonal diversity equal to or greater than 1E+08 were obtained and phages were produced ensuring presentation of antibody diversity.

### Example 8: Selections

Panning selections were performed as follows: Per selection condition the respective antigen for selection was blocked in microcentrifuge tubes using 10% BSA/PBST (Bovine Serum Albumin in Phosphate Buffer Saline with 0.1%Tween 20) and incubated with library phage derived from the phagemid vector pASF103 (approximately 1E+11 phage particles per selection condition). PBST comprises 137 mM NaCl, 2.7 mM KCl, 10 mM Na2HPO4, 1.8 mM KH2PO4 and 0.1%(w/v) Tween^{®} 20 detergent. Washes were performed using 10% BSA/PBST and phage were eluted using 0.25 mg/ml trypsin. Four selection rounds were performed and enrichments were observed against an irrelevant library used under the same conditions.

Individual colonies of *E. coli* TG1 cells infected with selected eluted phage pools obtained after the fourth round were picked into 96-well plates, master plates (MP) containing 100 µl of 2xTY medium containing 2% glucose and 100 µg/ml carbenicillin per well, and grown overnight at 37°C. MPs were stored in 20% glycerol at -80°C and used for periplasmic extract production, screening, and sequencing.

Statements (features) and embodiments of the methods, oligonucleotides, and compositions as disclosed herein are set here below. Each of the statements and embodiments as disclosed by the invention so defined may be combined with any other statement and/or embodiment unless clearly indicated to the contrary. In particular, any feature indicated as being preferred or advantageous may be combined with any other feature or features indicated as being preferred or advantageous.

## Claims

1. A method for constructing a VHH antibody library comprising the steps of
a) providing a cDNA preparation derived from an RNA extract from peripheral blood mononuclear cells of a *Camelidae* species; and
b) performing DNA amplification of VHH-CH2 encoding regions present in the cDNA preparation, whilst blocking DNA amplification of VH-CH1-CH2 encoding regions present in the cDNA preparation,
(i) wherein DNA amplification of VHH-CH2 encoding regions is initiated by a primer pair comprising a forward and a reverse primer, where the forward primer hybridizes to and/or upstream of the FR1 domain of the VHH-CH2 and VH-CH1-CH2 encoding regions and where the reverse primer hybridizes to and/or downstream of the CH2 domain of the VHH-CH2 and VH-CH1-CH2 encoding regions, thereby providing VHH-CH2 encoding amplicons;
(ii) wherein DNA amplification of VH-CH1-CH2 encoding regions is blocked by at least one blocking oligonucleotide.

2. The method according to claim 1, wherein the blocking oligonucleotide hybridizes to the CH1 domain of the VH-CH1-CH2 encoding region.

3. The method according to claim 1 or 2, wherein the blocking oligonucleotide hybridizes to the CH1 domain according to the polynucleotide of any of SEQ ID NO: 6 to 15, or a polynucleotide with 90%, 91%, 92%, 93%, 94%, 95%, 96%, 97%, 98% or 99% identity thereto.

4. The method according to any one of claims 1 to 3, wherein the VH-CH1-CH2 encoding region is blocked by two or more blocking oligonucleotides, wherein at least one blocking oligonucleotide hybridizes to the sense strand of the CH1 domain and at least one blocking oligonucleotide hybridizes to the anti-sense strand of the CH1 domain.

5. The method according to any one of 1 to 4, wherein the blocking oligonucleotide is a locked nucleic acid or a peptide nucleic acid.

6. The method according to any one of claims 1 to 5, wherein DNA amplification is performed using a polymerase lacking 5'-3' and/or 3'-5' exonuclease activity.

7. The method according to any one of claims 1 to 6, wherein the blocking oligonucleotide has a length of between 10 and 100 nucleotides or between 10 and 50 nucleotides, or between 15 and 30 nucleotides, or between 18 and 25 nucleotides.

8. The method according to any one of claims 1 to 7, wherein the at least one blocking oligonucleotide comprises a nucleotide sequence according to SEQ ID NOs: 1 or 2.

9. The method according to any one of claims 1 to 8, wherein the DNA amplification of VHH-CH2 encoding regions is initiated by a primer pair comprising at least two forward primers and a reverse primer.

10. The method according to any one of claims 1 to 9, wherein the forward primer, or the at least two forward primers, comprises a nucleotide sequence according to SEQ ID NOs: 3 and/or 4 and where the reverse primer comprises a nucleotide sequence according to SEQ ID NO: 5.

11. The method according to any one of the previous claims, wherein the method further comprises DNA amplification of VHH antibody encoding regions present in the VHH-CH2 encoding amplicons, thereby providing VHH antibody encoding amplicons.

12. The method according to any one of the previous claims, wherein the method further comprises introducing the amplicons into a suitable vector, preferably a phagemid vector.

13. The method according to any one of the previous claims, wherein the Camelidae species has been immunized prior to obtaining peripheral blood mononuclear cells therefrom.

14. A kit of parts comprising
- a primer pair suitable to amplify a VHH-CH2 and VH-CH1-CH2 encoding regions in a cDNA preparation derived from an RNA extract from peripheral blood mononuclear cells of a *Camelidae* species;
the primer pair comprising a at least one forward and a reverse primer, where the forward primer has the ability to hybridize to and/or upstream of the FR1 domain of the VHH-CH2 and VH-CH1-CH2 encoding regions and where the reverse primer has the ability to hybridize to and/or downstream of the CH2 domain of the VHH-CH2 and VH-CH1-CH2 encoding regions; and
- a blocking oligonucleotide that is adapted to block DNA amplification of the VH-CH1-CH2 encoding regions by the primer pair.

15. A method of obtaining one or more VHH binding to an antigen, comprising the steps of
(i) providing the VHH antibody library according to any one of claims 1 to 13 or the VHH antibody library obtained using the kit of parts of claim 14,
(ii) expressing said VHH antibody library,
(iii) screening said VHH antibody library for one or more VHH binding to said antigen, and
(iv) isolating said one or more VHH antibody;
whereby the *Camelidae* species was immunized with said antigen prior to obtaining peripheral blood mononuclear cells therefrom;. and where optionally the screening of said VHH antibody library for one or more VHH antibody binding to said antigen is performed using phage display.
